Europäisches Patentamt

(19) European Patent Office

Office européen des brevets

(11) Publication number: **0 037 603**
**B1**

(12) EUROPEAN PATENT SPECIFICATION

(45) Date of publication of patent specification: **15.02.84**   (51) Int. Cl.³: **C 07 D 207/267**

(21) Application number: **81200331.7**

(22) Date of filing: **25.03.81**

(54) Method for the preparation of N-methyl or N-ethyl-2-pyrrolidone.

(30) Priority: **04.04.80 NL 8002019**

(43) Date of publication of application:
**14.10.81 Bulletin 81/41**

(45) Publication of the grant of the patent:
**15.02.84 Bulletin 84/7**

(84) Designated Contracting States:
**BE DE FR GB IT NL**

(56) References cited:

**JOURNAL OF ORGANIC CHEMISTRY, vol. 41,
no. 4, 1976, pages 725-726 Washington, U.S.A.
J. AUERBACH et al.: "N-methylation of amides,
lactams, and ureas"**

(73) Proprietor: **STAMICARBON B.V.
Postbus 10
NL-6160 MC Geleen (NL)**

(72) Inventor: **Meyer, Peter Johan Nicolaas
Burg. Luytenstraat 31
NL-6151 GE Munstergeleen (NL)**
Inventor: **Penders, Josef Maria
Cuyleborg 141
NL-6228 BD Maastricht (NL)**

(74) Representative: **Pinckaers, August René et al,
OCTROOIBUREAU DSM Postbus 9
NL-6160 MA Geleen (NL)**

Courier Press, Leamington Spa, England.

### Method for the preparation of N-methyl or N-ethyl 2-pryrrolidone

The invention relates to a method for the preparation of N-methyl- or N-ethyl- 2-pyrrolidone by reduction of the corresponding N-(α-hydroxyalkyl)-2-pyrrolidone.

In Journal of Organic Chemistry, volume 41, no. 4 (1976), pages 725—726, two methods are described for the reduction of N-hydroxymethylpyrrolidone to N-methylpyrrolidone. In applying the one method, the reduction is performed with triethylsilane in the presence of trifluoroacetic acid and in applying the other, with hydrogen and palladium on carbon as a catalyst and again in the presence of trifluoroacetic acid. Owing to the presence of the trifluoroacetic acid, an acyliminium ion is formed probably from the hydroxy-methylpyrrolidone, which acyliminium ion is reduced to the N-methyl compound.

In Synthetic Communications 7 (8), 549—552 (1977) a third method is described for the performance of the said reduction, viz. the reduction with cyanoborohydride in trifluoroacetic acid.

A major objection of the said methods is the necessary use of trifluoroacetic acid. The applications of such an acid requires, in practice, a device of a very costly material. Furthermore, the reductants triethylsilane and cyanoborohydride applied are rather unsuitable for practical application, while, in view of the low yield per gramme of palladium according to the disclosure in said publication of Journal of Organic Chemistry, the use of the reductant hydrogen with palladium on carbon as catalyst will involve high catalyst costs.

It has now been found that the reduction of the said corresponding N-(α-hydroxyalkyl)-2-pyrrolidone can be very well effected at lower cost for reductant and without trifluoroacetic acid.

The method according to the invention for the preparation of N-methyl or N-ethyl substituted 2-pyrrolidone by reduction of the corresponding N-(α-hydroxyalkyl)-2-pyrrolidone with hydrogen and palladium on carbon as catalyst, is characterized in that the reduction is carried out in the liquid phase, in absence of trifluoroacetic acid, at a temperature of 50—125°C, a partial hydrogen pressure of 25—125 bar and in the presence of water or the product to be prepared as distributing agent.

N-hydroxymethyl-2-pyrrolidone can be obtained very suitable in the manner described in US patent specification 3,073,843, in which process the pyrrolidone is reacted with formaldehyde in the presence of a strong alkaline compound as catalyst, for instance potassium hydroxide. N-(α-hydroxyethyl)-2-pyrrolidone can, for instance, be prepared (see Brennstoff-Chemie Nr. 4 Bd. 48 pages 136—139) by reaction of acetaldehyde with the pyrrolidone in the presence of an acid catalyst, for instance water-free hydrogen chloride.

The method according to the invention can be applied continuously or discontinuously in different ways, for instance in a stirred reactor system, a loop reactor or a trickle phase reactor. If it is desired to prepare N-methyl-2-pyrrolidone according to the invention and if the hydroxymethyl pyrrolidone prepared by addition of formaldehyde to the pyrrolidone by means of a catalytic quantity of strong alkali is started from, the reaction mixture obtained in performing this addition can be applied as starting product without further purification. If desired, the distributing agent to be applied in performing the reduction according to the invention can in that case be present already when performing the addition of the formaldehyde to the pyrrolidone.

The reaction mixture obtained in performing the reduction according to the invention can be worked up as known in the art, for instance by distillation, while recovering the desired product and possibly non-converted starting compound.

The products obtained in applying the method according to the invention can be used as solvent, for instance for polyurethanes, and as a selective extracting agent in the petroleum industry.

In the following examples the invention is further elucidated.

### Example I

An autoclave of a capacity of 2 litres, provided with a stirrer and a heating jacket, is filled with 1000 g of water, 32 g of palladium on carbon catalyst (5% by weight of Pd) and 100 g of unpurified N-hydroxymethylpyrrolidone (prepared from 73.8 g of pyrrolidone, 26 g of paraformaldehyde and 0.2 g of KOH in the manner as described in the US-patent specification 3,073,843). The autoclave is closed and flushed with nitrogen. Subsequently, hydrogen is pressed into the autoclave to a pressure of 100 bar, upon which, during vigorous stirring, the mixture in the autoclave is heated to 100°C. The hydrogen pressure is then about 100 bar. The stirring is continued at this temperature till no more hydrogen is taken up, which is the case after about 30 minutes. The reaction mixture obtained is rapidly cooled and analyzed gas-chromatographically. This analyses shows that 96% of the N-hydroxymethylpyrrolidone is converted, and that the yield of N-methylpyrrolidone amounts to 95% of the yield theoretically possible.

### Example II

Example I is repeated with starting product purified by recrystallization from toluene and with 24 g of the palladium catalyst. Instead of 1000 g of water, 1000 g of N-methylpyrrolidone is used as distributing agent. It now takes 90 minutes before the take-up of hydrogen causes.

The conversion is 94% and the yield 87% of the yield theoretically possible.

### Example III

An autoclave as described in example I is filled with 500 g of formalin (37% by weight of formaldehyde) and a mixture of 524 g pyrrolidone and 29 g of potassium carbonate. The autoclave is closed, flushed with nitrogen and subsequently, during stirring, heated for 2 hours at 80°C. After that 124 g of palladium on carbon catalyst (2% by weight of Pd) is pressed, via a dosing vessel, into the autoclave, and the pressure is brought, with hydrogen, to 100 bar. While being stirred, the reaction mixture is subsequently, for 120 minutes, kept at a temperature of 80°C. No more hydrogen is then taken up. After cooling the reaction mixture obtained is analyzed. 415.5 g of N-methylpyrrolidone is obtained. The conversion of the pyrrolidone amounts to 90%. Of the converted pyrrolidone 75.6% has been converted into the desired product.

### Example IV

An autoclave as in Example I is filled with 1000 g of water, 26 g of palladium on carbon catalyst (2% by weight of Pd) and 100 g of N-($\alpha$-hydroxyethyl)-pyrrolidone. The autoclave is closed and flushed with nitrogen. Subsequently, hydrogen is pressed into the autoclave to a pressure of 50 bar, and during vigorous stirring the mixture is heated up to 100°C. While being stirred, the mixture in the autoclave is subsequently, for 60 minutes, kept at a temperature of 100°C. The hydrogen take-up has then stopped. After cooling, the reaction mixture obtained is analyzed. This analysis shows that the starting product is fully converted and the yield of N-ethylpyrrolidone is 99.2% of the yield theoretically possible.

## Claims

1. Method for the preparation of N-methyl or N-ethyl substituted 2-pyrrolidone by reduction of the corresponding N-($\alpha$-hydroxyalkyl)-2-pyrrolidone with hydrogen and palladium on carbon as catalyst, characterized in that the

### Patentansprüche

1. Verfahren zur Herstellung von N-Methyl- oder N-Ethyl- substituiertem 2-Pyrrolidon durch Reduktion des korrespondierenden N-($\alpha$-Hydroxyalkyl)-2-Pyrrolidons mit Wasserstof und Palladium auf Kohle als Katalysator, dadurch gekennzeichnet, dass die Reduktion in der Flüssigphase durchgeführt wird, und zwar in Abwesenheit von Trifluoressigsäure, bei einer Temperatur von 50—125°C, einem Wasserstoff-Partialdruck von 25—125 bar und in Anwesenheit von Wasser oder von dem zu bereitenden Produkt als Verteilungsagens.

2. Verfahren nach Anspruch 1 zur Herstellung von N-Methyl-2-Pyrrolidon, dadurch gekennzeichnet, dass von einem ungereinigten Reaktionsgemisch, erhalten durch Hinzufügung von Formaldehyd zum Pyrrolidon unter dem Einfluss einer katalytischen Quantität eines starken Alkalis ausgegangen wird.

### Revendications

1. Procédé de préparation d'une 2-pyrrolidone N-méthyl- ou N-éthyl-substituée par réduction de la N-($\alpha$-hydroxyalkyl)-2-pyrrolidone correspondante avec de l'hydrogène et du carbone palladié à titre de catalyseur, caractérisé en ce qu'on effectue la réduction en phase liquide, en l'absence d'acide trifluoracétique, à une température de 50 à 125°C, sous une pression partielle d'hydrogène de 25 à 125 bars et en présence d'eau ou du produit à préparer à titre d'agent distributeur.

2. Procédé selon la revendication 1 pour la préparation de N-méthyl-2-pyrrolidone, caractérisé en ce qu'on part du mélange de réaction non purifié obtenu par addition du formaldéhyde à la pyrrolidone sous l'effet d'une quantité catalytique d'un alcali fort.

reduction is carried out in the liquid phase, in absence of trifluoroacetic acid, at a temperature of 50—125°C, a partial hydrogen pressure of 25—125 bar and in the presence of water or the product to be prepared as distributing agent.

2. Method according to claim 1 for the preparation of N-methyl-2-pyrrolidone, characterized in that an unpurified reaction mixture obtained by addition of formaldehyde to the pyrrolidone under the influence of a catalytic quantity of strong alkali is started from.